(19) Europäisches Patentamt / European Patent Office / Office européen des brevets

(11) **EP 2 792 377 A1**

(12) **EUROPEAN PATENT APPLICATION**

(43) Date of publication:
**22.10.2014 Bulletin 2014/43**

(51) Int Cl.:
*A61M 1/16* (2006.01)    *A61M 1/36* (2006.01)

(21) Application number: **13163728.2**

(22) Date of filing: **15.04.2013**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventors:<br>• **Bene, Bernard**<br>  **69540 Irigny (FR)**<br>• **Vantard, Georges**<br>  **38090 Villefontaine (FR)** |
| (71) Applicant: **Gambro Lundia AB**<br>**220 10 Lund (SE)** | (74) Representative: **Ponzellini, Gianmarco et al**<br>**Ponzellini, Gioia e Associati S.r.l.**<br>**Via Mascheroni, 31**<br>**20145 Milano (IT)** |

(54) **Medical infrastructure and medical monitoring apparatus for surveillance of patients over a plurality of extracorporeal blood treatment sessions**

(57)    A medical monitoring apparatus and a method for surveillance of patients submitted to a plurality of extracorporeal treatment sessions executed by at least one extracorporeal blood treatment machine is disclosed. An infrastructure including a plurality of blood treatment machines and the monitoring apparatus is also disclosed. The monitoring apparatus and the method are configured for determining presence of peaks in the values taken over time by a characteristic $(Cd(t))$ of the dialysis liquid during a same treatment session and for determining presence of peaks in the values taken over time by the parameter $(BV\%(t))$ related to blood volume change during the same treatment session. Based on the detection of the above peaks the apparatus and method verify presence and cause of a condition of recirculation.

FIG.1

EP 2 792 377 A1

**Description**

The present invention relates a medical infrastructure and to medical monitoring apparatus for surveillance of patients, such as for example patients affected by kidney failure or liver failure, over a plurality of extracorporeal treatment sessions during which blood is withdrawn from a patient via a blood removal line, is treated in a blood treatment unit and then is returned to the patient via a blood return line. The invention is directed to monitor a plurality of patients over a time frame such that an operator, e.g., a medical doctor, may be able to more easily survey his patients. The invention may serve for instance to timely identify those patients showing vascular access problems and who may need a treatment prescription modification or more detailed exams.

**Background**

Treatments such as, for example, hemodialysis, hemofiltration, hemodiafiltration, plasmapheresis, blood component separation, blood oxygenation, require that blood is removed from a blood vessel at a vascular access and returned to the same or other blood vessel. In hemodialysis, for example, a blood access may be surgically created in the nature of an arterio-venous shunt, commonly referred to as a fistula. Blood needles are inserted in the fistula: blood is taken out from the fistula via a needle at an upstream position and blood is returned to the fistula via a needle at a downstream position. The arterio-venous shunt or fistula is a blood access having capability of providing a high blood flow and being operative during several years. It is surgically created by connecting, for example, the radial artery to the cephalic vein at the level of the forearm. The venous limb of the fistula thickens during the course of several months, permitting repeated insertion of dialysis needles. An alternative blood access may be the arterio-venous graft, in which a connection is generated between the radial artery at the wrist and the basilic vein. The connection is made with a tube graft made from e.g. autogenous saphenous vein or from polytetrafluorethylene (PTFE, Teflon). The needles are inserted in the graft. A further example of a blood access is a dual-lumen catheter surgically implanted into one of the large veins. Further types of blood access devices may find use in specific situations.

In order for extracorporeal blood treatment apparatus to efficiently deliver blood treatment, it is important that the vascular access device (such as a needle or catheter) is properly positioned and that the patient fistula or other access is properly functioning. A non properly working vascular access (such as for example a clogged fistula) may cause reduction in the access flow and lead to recirculation, i.e. to partial or total recirculation into the extracorporeal blood circuit of already treated blood. Recirculation may also be caused by a wrongly positioned access device (for example needles placed in the reversed position).

More in detail, during hemodialysis for instance, it is desirable to obtain a blood flow rate in the range of 150 - 500 ml/min in the extracorporeal circuit. Thus, the vascular access must be prepared for delivering such flow rates. In the absence of a sufficient blood flow in fistula or in case of wrong positioning of the needles, the extracorporeal circuit blood pump takes up some of the already treated blood entering the fistula causing the so called access or fistula recirculation, leading to poor treatment results and progressive reduction of treatment efficiency.

A common cause of poor flow with AV fistulas is partial obstruction of the venous limb due to fibrosis secondary to multiple venipunctures or due to stenosis. Proper detection of access flow reduction may help in carrying out maintenance procedures on the access thereby avoiding any access failure.

Various techniques have been developed in the past for monitoring the status of the fistula or other vascular access.

A non-invasive technique that allows measurement of flow through AV fistulas and grafts is color Doppler ultrasound. Magnetic Resonance Imaging (MRI) has also been used. However, these techniques require expensive equipment and are not easily used in the dialysis clinic environment.

Methods have been suggested for monitoring recirculation and access flow involving injection of a marker substance in blood, and, subsequently, detecting presence of the marker in the blood withdrawn from the patient. These methods normally involve measurement of a property in the blood circulating in the extracorporeal blood circuit. Examples of such methods can be found in US 5,685,989, US 5,595,182, US 5,453,576, US 5,510,716, US 5,510,717, US 5,312,550. These methods have the disadvantage that they require injection of a marker substance and use of dedicated equipment for the measurements.

EP 928 614 and WO 00/24440 disclose methods for detecting fistula flow and recirculation using a measure of a post dialyzer concentration of a substance, in particular urea, in the effluent fluid. This measure is taken before and after a flow reversal of the blood lines connected to the vascular access. These methods require equipment for measuring the urea concentration and dedicated devices for reversing the blood lines.

WO2008/087470 discloses a control system for the determination of the state of a vascular access of a patient intended to follow successive sessions of extracorporeal blood treatment by extraction and return of the blood via the vascular access; the system has means for determining the value of at least one hemodynamic extracorporeal parameter of the patient for at least two sessions and means for determining the value of the purification effectiveness of the treatment for at least two sessions; a risk score relating to the state of the vascular access of the patient is then determined as a function of values of the hemodynamic extracorporeal parameter and values of the purification effectiveness.

Finally, a study published in 2006 [Sullivan, Mercadal, Coevoet, Albadawy, Hacini, Bene, Deray, Petitelerc, entitled "Analysis of the optical concentration curve to detect access recirculation", Kidney Int. 2006;69(4)769-7] demonstrated that presence of a spike of the optical blood volume curve occurring during the dialysance measurement may be indicative of access recirculation.

## Summary of the invention

In this situation, it is an object of the invention to offer a technical solution arranged to easily gather information relating to a patient in order to reliably determine whether recirculation is present.

An auxiliary object is that of providing a solution allowing the determination of the cause for recirculation.

Another object is that of offering a technical solution capable of determination of whether recirculation is caused by deterioration of the vascular access, e.g. fistula stenosis, or wrong positioning of the access device(s), e.g. detection of needles installed in the reversed position.

Moreover, it is one object finding a solution aranged to be easily integrated with other methods for monitoring the status of the vascular access.

Additionally, it is an auxiliary object finding a solution not requiring dedicated hardware equipment to be added to the conventional architecture of dialysis machines.

At least one of the above objects is substantially reached by an by a process and by an apparatus according to one or more of the appended claims.

Aspects of the invention are here-below disclosed.

A first aspect concerns a medical monitoring apparatus for surveillance of patients submitted to a plurality of extracorporeal treatment sessions executed by at least one extracorporeal blood treatment machine; the monitoring apparatus comprises:

at least one memory block configured for storing:

- measured values of a parameter (BV%(t)) relating to the change of blood volume, in the blood circulating in an extracorporeal blood circuit of said machine, as measured during the course of blood treatment, said measured values of said parameter relating to a same patient and referring to a plurality of distinct treatment sessions;
- measured or set values of a physical or chemical characteristic (Cd(t)) of the dialysis liquid as set or, respectively, as measured during the course of the extracorporeal blood treatment, said values of said characteristic relating to said same patient and referring to said plurality of distinct treatment sessions;

at least a control unit connected to the memory block and configured for:

- determining presence of peaks in the values taken over time by the characteristic (Cd(t)) during a same treatment session;
- determining presence of peaks in the values taken over time by the parameter (BV%(t))during the same treatment session;
- checking whether - in presence of a peak in the values of the characteristic - there is a corresponding presence of a peak in the values of the parameter thereby defining a pair of substantially concurrent peaks in a same treatment session;
- determining whether presence of said pair of substantially concurrent peaks in a same treatment session takes place for at least two - optionally at least three - consecutive peaks in the values of the characteristic, and
- if presence of said pair of substantially concurrent peaks occurs for at least two - optionally at least three - consecutive peaks in the values of the characteristic, then establishing that a condition of recirculation took place in the treatment session;
- verifying if said condition of recirculation is taking place on the same patient for at least two consecutive treatment sessions;
- identifying a cause of recirculation based on the outcome of said verification on said condition of recirculation.

In a 2nd according to the 1st aspect, the medical monitoring apparatus further comprises a communication interface connected to the control unit.

In a 3rd aspect according to the preceding aspect, the control unit is configured for establishing a communication with a control device of the extracorporeal blood treatment machines via said communication interface.

In a 4th aspect according to any one of the preceding two aspects, the control unit of the monitoring apparatus is configured for:

- receiving measured or set values of said characteristic (Cd(t)), said values of said characteristic relating to the same patient and referring to a plurality of distinct treatment sessions;
- checking presence of peaks in the characteristic (Cd(t)) by referring to either the measured or the set values of said characteristic (Cd(t)).

In a 5th aspect in accordance with the 4th aspect, identifying a cause of recirculation comprises:

- establishing that the cause of recirculation is a degradation in the status of the vascular access, optionally a fistula stenosis or fibrosis, if said condition of recirculation is verified to take place for at least two consecutive treatment sessions on the same patient.

In a 6th aspect according to any one of the preceding two aspects, identifying a cause of recirculation comprises:

- establishing that the cause of recirculation is due to improperly positioned access devices, optionally access devices installed in reversed position in the vascular access, if said condition of recirculation is verified to take place for one isolated treatment session immediately preceded and followed by treatment sessions where said condition of recirculation is verified not to take place.

In a 7th aspect according to any one of the preceding three aspects, said step of checking comprises verifying if each pair of substantially concurrent peaks comprises peaks in opposite in direction with respect to the respective baseline values.

In a 8th aspect according to any one of the preceding four aspects, the control unit is configured for establishing that a condition of recirculation took place in the treatment session if:

- presence of said pair of substantially concurrent peaks occurs for at least two consecutive peaks in the same session and
- the magnitude of the two consecutive peaks in the parameter values is substantially constant.

In a 9th aspect according to any one of the preceding five aspects, the control unit is configured for:

- in-vivo determining, at each peak in the characteristic (Cd(t)), values of a parameter (D(t)) relative to efficiency of the dialysis treatment,
- comparing the values of the efficiency parameter (D(t)) against a respective threshold,
- concluding that a condition of recirculation took place in the treatment session if:

    presence of a sequence of consecutive pairs of substantially concurrent peaks is detected and the in-vivo determined value of the efficiency parameter, relating to one or both consecutive peaks in the characteristic, is below said prefixed threshold.

In a 10th aspect it is provided an extracorporeal blood treatment machine comprising the medical monitoring apparatus according to anyone of preceding aspects.
In an 11th aspect it is a provided a medical infrastructure comprising:

    at least one extracorporeal blood treatment machine having:

- an extracorporeal blood circuit with a blood treatment unit provided with at least a first chamber and at least a second chamber separated from one another by a semipermeable membrane;
- a dialysate circuit comprising:

    a dialysis line configured to convey fresh dialysis fluid to the second chamber,
    a fluid evacuation line configured to discharge spent dialysis fluid exiting from the second chamber, and
    a fluid delivery section connected to the dialysis line and configured for adjusting at least one physical or chemical characteristic (Cd(t)) in the fresh dialysis fluid;

- at least one first sensor connected to the extracorporeal blood circuit and configured for measuring values of a parameter (BV%(t)) - relating to the change of blood volume in the blood circulating in the extracorporeal blood circuit - measured during the course of extracorporeal blood treatment;
- a control device connected to said first sensor and configured for receiving from said first sensor values taken over time by said parameter, the control device being also connected to said fluid delivery section and configured for controlling said fluid delivery section to adjust said characteristic Cd(t);

    a monitoring apparatus according to any one of the aspects from the 1st to the 9th.

In a 12th aspect according to the 11th aspect, the monitoring apparatus is remotely located and communicatively connected to the extracorporeal blood treatment machine; wherein the control unit is configured for establishing a communication with the control device of the extracorporeal blood treatment machine and for receiving said values of the parameter and of the characteristic from said control device.
In a 13th aspect according to any one of the preceding two aspects, the control device is configured for:

- causing fresh treatment liquid to flow in the dialysis line to the secondary chamber with said characteristic being at a baseline (Cdset) set value which is either constant or varying in a known manner over time;
- causing spent liquid to flow out of the second chamber into the evacuation line;
- causing a peak in the characteristic (Cd(t)) by imposing an upstream peak (Cdin) to said characteristic in the fresh treatment liquid with respect to said prescription baseline thereby determining a corresponding and timely delayed downstream peak (Cdout) of the same characteristic in the spent liquid flowing in the evacuation line.

In a 14th aspect according to any one of the preceding three aspects, the extracorporeal blood treatment machine comprises a second sensor configured for measuring values of the physical or chemical characteristic of the fresh dialysis liquid flowing in the dialysis line during the course of the extracorporeal blood treatment.

In a 15th aspect according to any one of the preceding four aspects, the extracorporeal blood treatment machine comprises a third sensor configured for measuring values of the physical or chemical characteristic of the spent liquid flowing in the evacuation line during the course of the extracorporeal blood treatment.

In a 16th aspect according to any one of the preceding two aspects, receiving measured values of said characteristic (Cd(t)) comprises receiving the values taken over time by said characteristic which comprises the conductivity or the concentration for at least one substance in the fresh or in the spent dialysis liquid.

In a 17th aspect according to any one of the preceding six aspects, said step of checking whether - in presence of a peak in the values of the characteristic - there is a corresponding presence of a peak in the values of the parameter also comprises verifying if each pair of substantially concurrent peaks are peaks in directed according to opposite directions with respect to the respective baseline values. In practice the two peaks, one in the characteristic and one in the parameter, shall be substantially concurrent (in reality the peak in the parameter is slightly delayed) and shall be oriented in opposite direction with respect to the respective baseline in order to define a pair of substantially concurrent peaks in a same treatment session.

In a 18th aspect according to any one of the preceding aspects, said step of determining presence of peaks in the values taken over time by the characteristic comprises:

- analyzing the values taken by said characteristic (Cd(t)) over time,
- identifying presence of a peak when the analyzed values of the characteristic (Cd(t)) show a sudden change with respect to the respective baseline, wherein a sudden change in the characteristic value is an increase or a decrease by at least 5% in the value of the characteristic taking place in a time interval of less than 5 minutes.

In a 19th aspect according to any one of the preceding aspects, said step of determining presence of peaks in the values taken over time by the parameter (BV%(t)) comprises:

- analyzing the values taken by said parameter over time,
- identifying presence of a peak if the analyzed values of the parameter show a sudden change in the parameter value (BV%(t) with respect to the respective baseline, wherein a sudden change is an increase or a decrease by at least 10% in the value of the parameter taking place in a time interval of less than 5 minutes.

In a 20th aspect it is provided a method of surveillance of patients submitted to a plurality of extracorporeal treatment sessions executed by at least one extracorporeal blood treatment machine, the method of surveillance comprising:

- storing measured values of a parameter (BV%(t)) relating to the change of blood volume in the blood circulating in an extracorporeal blood circuit of said machine as measured during the course of blood treatment, said measured values of said parameter relating to a same patient and referring to a plurality of distinct treatment sessions;
- storing measured or set values of a physical or chemical characteristic (Cd(t)) of the dialysis liquid as set or, respectively, as measured during the course of the extracorporeal blood treatment, said values of said characteristic relating to said same patient and referring to said plurality of distinct treatment sessions;
- determining presence of peaks in the values taken over time by the characteristic (Cd(t)) during a same treatment session;
- determining presence of peaks in the values taken over time by the parameter (BV%(t))during a same treatment session;
- checking whether - in presence of a peak in the values of the characteristic - there is a corresponding presence of a peak in the values of the parameter thereby defining a pair of substantially concurrent peaks in a same treatment session;
- determining whether presence of said pair of substantially concurrent peaks in a same treatment session takes place for at least two - optionally at least three - consecutive peaks in the values of the characteristic, and
- if presence of said pair of substantially concurrent peaks occurs for at least two - optionally at least three - consecutive peaks in the values of the characteristic, then establishing that a condition of recirculation took place in the treatment session;
- verifying if said condition of recirculation is taking place on the same patient for at least two consecutive treatment sessions;
- identifying a cause of recirculation based on the outcome of said verification on said condition of recirculation.

In a 21st aspect according to the preceding aspect, identifying a cause of recirculation comprises:

- establishing that the cause of recirculation is a degradation in the status of the vascular access, optionally a fistula stenosis or fibrosis, if said condition of recirculation is verified to take place for at least two consecutive treatment sessions on the same patient, and
- establishing that the cause of recirculation is due to improperly positioned access devices, optionally access devices installed in reversed position in the vascular access, if said condition of recirculation is verified to take place for one isolated treatment session immediately preceded and followed by treatment sessions where said condition of recirculation is verified not to take place.

In a 22nd aspect according to any one of the preceding two aspects, said step of checking comprises verifying if each pair of substantially concurrent peaks comprises peaks in opposite in direction with respect to the respective baseline values.

In a 23rd aspect according to any one of the preceding three aspects, it is established that a condition of recirculation took place in the treatment session if:

- presence of said pair of substantially concurrent peaks occurs for at least two consecutive peaks in the same session and
- the magnitude of the two consecutive peaks in the parameter values is substantially constant.

In a 24th aspect according to any one of the preceding four aspects, the method comprises:

- in-vivo determining, at each peak in the characteristic (Cd(t)), values of a parameter (D(t)) relative to efficiency of the dialysis treatment,
- comparing the values of the efficiency parameter (D(t)) against a respective threshold,
- concluding that a condition of recirculation took place in the treatment session if:

  presence of a sequence of consecutive pairs of substantially concurrent peaks is detected and the in-vivo determined value of the efficiency parameter, relating to one or both consecutive peaks in the characteristic, is below said prefixed threshold.

## Description of the drawings

The following drawings relating to aspects of the invention are provided by way of non limiting example:

- figure 1 is a schematic illustration of an infrastructure of monitoring a plurality of patients undergoing extracorporeal blood treatment,
- figure 2 shows an exemplary blood treatment machine which may be part of the infrastructure of figure 1;
- figures 3A and 3B show a diagram of percentage variation of the blood volume BV%(t) in the blood circulating in extracorporeal blood circuit from start of treatment and, respectively, of the conductivity Cd(t) as measured in the spent dialysate, in a case where there is no recirculation detected;
- figures 4A and 4B show a diagram of BV%(t) and, respectively, of Cd(t), analogous to the diagram of figures 3A and 3B, in a case where recirculation is detected;
- figures 5A and 5B show a diagram of BV%(t) and, respectively, of Cd(t), analogous to the diagram of figures 3A and 3B, in a further case where recirculation is detected;
- figure 6 is a schematic illustration of a graphic user interface of a medical monitoring apparatus part of the infrastructure of figure 1 showing BV%(t), Cd(t) and dialysance D(t), wherein A represents the duration of an induced recirculation, B a spike or peak in the BV%(t) curve, C a decrease of dialysance during the induced recirculation, D a step in the characteristic Cd(t) during the dialysance measurement;
- figure 7 is a flowchart of a process of monitoring a plurality of patients undergoing extracorporeal blood treatment according to aspects of the invention.

## Detailed description

### Infrastructure

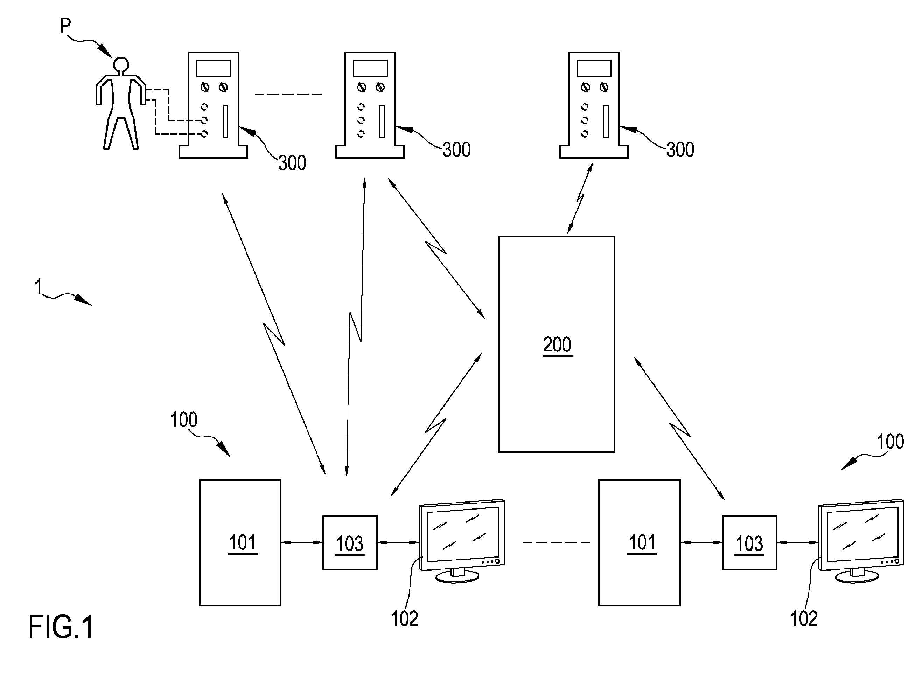

With reference to the appended drawing tables, and particularly referring to the exemplifying configuration represented in figure 1, reference numeral 1 indicates a medical infrastructure which is for example configured for monitoring a plurality of patients P affected by kidney failure over a time frame T covering a plurality of separate treatment sessions of each patient. Patients suffering from chronic kidney insufficiency are treated 2 to 4 times per week with extracorporeal blood treatments which may last from 3 to 5 hours each: the infrastructure 1 surveys a plurality of blood treatment machines 300 which may be used for the treatment of the monitored patients P. The blood treatment machines 300 are communicatively connected to one or more monitoring apparatus 100. The communication may be a direct communication, for instance a wireless communication or a wired communication, or the communication may take place via at least one intermediate unit 200, such as a server device, which collects data from the blood treatment machines 300 and sends the collected data to the monitoring apparatus 100. Of course, a number of intermediate units may be used depending upon the circumstances: the architecture of the communication network between the blood treatment machines 300 and the monitoring apparatus 100 may be of any type suitable to allow the described interactions.

## The blood treatment machines

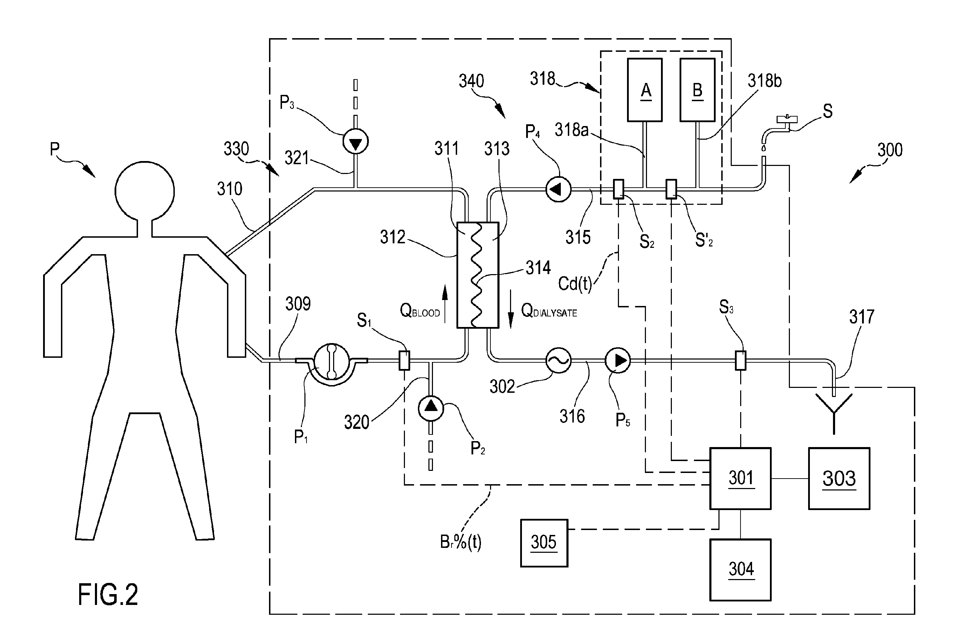

As shown if figure 2, each blood treatment machine 300 includes a treatment unit 312, such as for example a dialyzer or an hemofilter or an hemodiafilter, or a plasmafilter, provided with at least a first chamber 311 and with at least a second chamber 313 separated from one another by a semipermeable membrane 314. A blood removal line 309 is connected to an inlet port of the first chamber 311 and is configured to remove blood from a patient, while a blood return line 310 is connected with an outlet port of the first chamber 311 and configured to return treated blood to the patient. The blood removal line, the blood return line and the first chamber are part of an extracorporeal blood circuit 330; a blood pump $P_1$ - for example a peristaltic pump - may be operative on the extracorporeal blood circuit, e.g. in correspondence of the blood removal line 309 (as shown in figure 1) or in correspondence of the blood return line 310 to cause circulation of blood in the extracorporeal circuit at a desired blood flow rate ($Q_{BLOOD}$ in fig.1). The extracorporeal blood treatment machine also comprises a dialysate circuit 340 which is designed to deliver, fresh dialysis fluid to the blood treatment unit at a desired flow rate ($Q_{DIALYSATE}$ in fig.1) and to discharge to a waste the spent dialysis fluid and the ultrafiltrate coming from the second chamber 313 of the treatment unit 312. The dialysate circuit 340 comprises a dialysis line 315 connected to the inlet port of the second chamber 313 and configured to convey fresh dialysis fluid to the second chamber; the dialysate circuit also includes a fluid evacuation line 316 connected to an outlet port of the second chamber 313 and configured to discharge spent dialysis fluid exiting from the second chamber. The dialysis line and the evacuation line may include a respective pump $P_4$ and $P_5$ for circulating fluid through the dialysate circuit. This latter may also comprise a fluid delivery section 318 connected to the dialysis line 315 and configured for adjusting at least one physical or chemical characteristic Cd(t) in the fresh dialysis fluid: for instance the preparation section may receive pure or ultrapure water from a water source S and may add the electrolytes, using appropriate delivery devices schematically represented with block A, and buffer substances, using appropriate delivery devices schematically represented with block B, to the water which is thus transformed into a dialysis fluid. Note that, alternatively, the fluid delivery section may include one or more containers of pre-confectioned dialysis fluid and at least one means (such as a concentrate line) for changing the concentration or the conductivity of the fresh dialysis fluid.

In accordance with an optional aspect, the blood treatment machines 300 may also include one or more pre-dilution lines 320 with respective pump $P_2$ for delivering a replacement fluid or other fluid into the blood withdrawal line 309, and one or more post-dilution lines 321 with respective pump $P_3$ for delivering a replacement fluid or other fluid into the blood return line 310.

Pumps $P_1$, $P_2$, $P_3$, and $P_4$ and the fluid preparation section 318 are connected to and controlled by a control device 301 which is part of the apparatus 300 and which is also connected to the pumps described above and to other actuators and sensors which will be described herein below. The control device 301 may comprise a digital processor (CPU) with associated memory block (or memories) 303, an analogical type circuit, or a combination of one or more digital processing units with one or more analogical processing circuits. In the present description and in the claims it is indicated that the control device 301 is "configured" or "programmed" to execute certain steps: this may be achieved in practice by any means which allow configuring or programming the control device. For instance, in case of a control device comprising one or more CPUs, one or more programs are stored in an appropriate memory: the program or programs contain instructions which, when executed by the control device 301, cause the control device to execute the steps herein described and/or claimed in connection with the control device. Alternatively, if the control device 301 is of an analogical type, then the circuitry of the control device is designed to include circuitry configured, in use, to process electric signals such as to execute the steps herein disclosed or claimed. The control device 301 is also configured for allowing transmission of information to the intermediate unit 200 and/or to the monitoring apparatus 100.

The blood treatment machine 300 also comprises a first sensor $S_1$ located on the blood circuit and - for instance - positioned on the blood withdrawal line 309. The first sensor $S_1$ is configured for detecting, during execution of the extracorporeal blood treatment, the values of a parameter which is related to the variation of blood volume BV%(t). For instance, the parameter may be either the variation of blood volume BV%(t) or a parameter from which the variation in blood volume may be calculated in relation to the blood of a patient subjected to treatment. The first sensor or blood volume variation sensor $S_1$ may for example be an optical sensor, able to detect a variation in the optical properties of the blood crossing a calibrated portion of tube. For example, blood volume variation detection may comprise calculating, by control device 301 a percentage variation of the blood volume BV%(t) in the blood circulating in extracorporeal blood circuit from start of treatment based on the measurement of the concentration of hemoglobin in the blood, according to formula:

$$BV\%(t) = (HGB_0 / HGB_t) - 1,$$

where $HGB_0$ represents the concentration of hemoglobin at start of treatment and $HGB_t$ the concentration of he-

moglobin at time t in which BV%(t) is calculated.

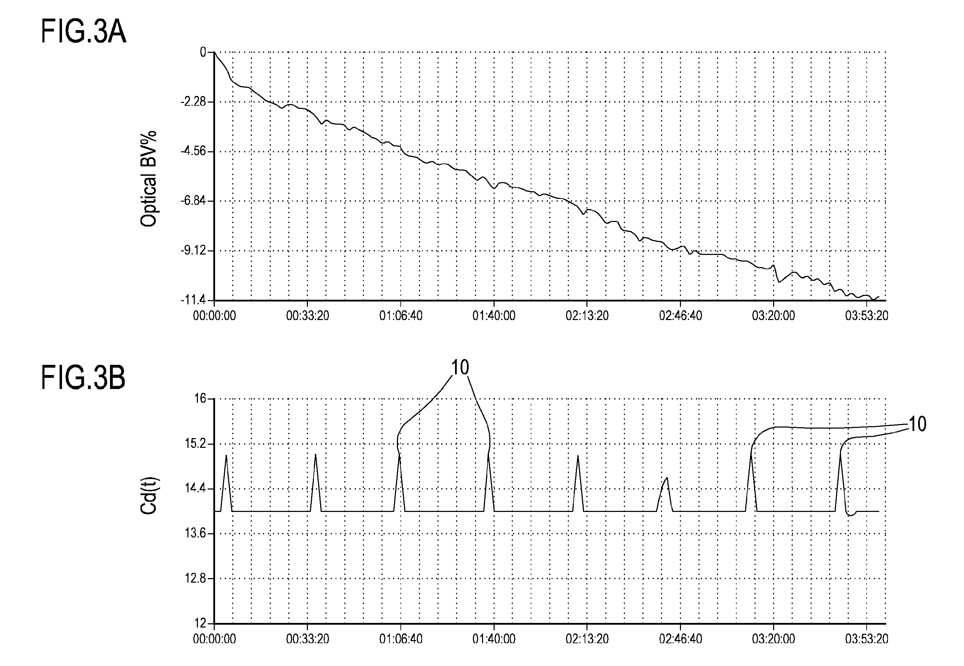

Values of BV%(t) as above calculated are represented in figures 3A, 4A, 5A.

The hemoglobin concentration may be calculated based on the variation of optic absorbance at a predetermined wavelength, detected by an optical sensor, of the blood flowing in the blood removal line 309. The optical sensor is for example associated to a tract of tube, e.g. positioned in the blood removal line 309, having the appropriate optical properties which have previously measured or which are known. Of course the values of HGB may alternatively be measured with techniques other than the one described above, such as measuring other blood properties (e.g.: capacitance, impedance) without departing from the scope of the present invention.

The machine 300 may further comprise at least one second sensor $S_2$ configured for detecting values of a physical or chemical characteristic Cd(t) of the fresh dialysis liquid flowing in the dialysis line, as measured during the course of the extracorporeal blood treatment. For example, the second sensor $S_2$ may be designed for detecting conductivity or sodium concentration (or concentration of another substance that is to be monitored) of the liquid flowing through the dialysis line 315. For example, the second sensor may be located immediately downstream of the fluid preparation section 318 which, inter alia, also has the task of regulating a composition of the dialysis liquid. In the example of figure 2, the fluid preparation section 318 comprises one or two containers of concentrate A, B (note that three or more containers of concentrate may also be envisaged depending on the design of the fluid preparation section) located on respective injection lines 318a, 318b which are configured to supply substances such as electrolytes, buffer agents or other towards the dialysis line 315. The concentrate containers may comprise concentrates in the liquid or solid state, for example powder. Injection pumps may be present on the injection lines to circulate fluid along the respective injection line towards line 315. The second sensor $S_2$ is able to provide the control device 301 with a signal e.g. relative to conductivity of the dialysis liquid or relative to the concentration of a predetermined substance (for example sodium $Na_D$) of the fluid exiting the fluid preparation section and entering into dialysis line 315. Note that, optionally, a further concentration or conductivity sensor $S_2'$ may be located in correspondence of segments of line 315 extending between the injection points of injection lines 318a and 318b. In case there would be more than two concentrate containers with respective independent injection lines connected to line 315, then further concentration or conductivity sensors could be envisaged. The control device 301 may act on the fluid preparation section 318 and in particular on the mentioned concentrate pumps in order to regulate the conductivity Cd or concentration, for example of sodium $Na_D$, of the liquid flowing into the dialysis line 315.

The machine 300 may further comprise a third sensor $S_3$ configured for detecting values of a physical or chemical characteristic Cd(t) of the used dialysis liquid flowing in the evacuation line 316, as measured during the course of the extracorporeal blood treatment. For example, the third sensor $S_3$ may be designed for detecting conductivity or sodium concentration (or concentration of another substance that is to be monitored) of the liquid flowing through the evacuation line 316.

The control device 301 is connected to the at least one, optionally two or more of said sensors. In detail, in accordance with one possible alternative, the apparatus comprises at least the first sensor $S_1$, which is connected to the control unit that uses set values imposed to the characteristic Cd(t). In a second alternative comprises at least the first sensor S1 and at least one of said second and third sensors $S_2$ and $S_3$, and the control unit is connected to the first sensor and to at least one of said second and third sensors and thus receives the measured values of the parameter BV%(t) and of the characteristic Cd(t) in the fresh dialysis fluid or in the spent dialysis fluid. In accordance with a particular embodiment the apparatus includes the first, second sensors $S_1$ and $S_2$, and the third sensor $S_3$ as well: in this case, the control unit is connected to the first sensor and to both said second and third sensors and thus receives the measured values of the parameter BV%(t) and of the characteristic Cd(t) in the fresh dialysis fluid and in the spent dialysis fluid. In case also the further sensor $S_2'$, then the control unit would be connected and receive signals from this sensor too.

Moreover, the control device 301 is configured to drive pumps $P_4$ and $P_5$ for causing fresh treatment liquid to flow in the line 315 to the second chamber and for causing spent liquid to flow out of the second chamber into the evacuation line 316. During normal treatment, the control device receives from the second sensor $S_2$ the values taken by the physical or chemical characteristic Cd(t) of the dialysis liquid flowing into the dialysis line 315 and controls said characteristic to be at a baseline $Cd_{set}$ set value which is either constant or varying in a known manner over time. The baseline $Cd_{set}$ set value may for instance be entered via a user interface 304 connected to the control device 301 and then be stored in a memory block 303 also connected to control device 301.

The control device 301 is configured to cause, for instance at time intervals acting on the fluid preparation section 318, an upstream peak $Cd_{in}$ to the characteristic in the fresh treatment liquid with respect to said prescription baseline thereby determining a corresponding and timely delayed downstream peak $Cd_{out}$ of the same characteristic in the spent liquid flowing in the evacuation line 316. Note that in a specific embodiment the control device 301 may be configured to cause the upstream peak at regular, i.e. periodic, time intervals.

For the purpose of the present invention peak in the characteristic Cd(t) means a variation in the characteristic with respect to the respective baseline value: the variation may be either an increase followed by a subsequent decrease bringing the characteristic value substantially back to the baseline value. The peak in the characteristic Cd(t) may include a variation lasting from few seconds to 10 minutes: for instance the peak could be a substantially instantaneous pulse-like perturbation lasting for 10 or more seconds or it could be a change that is maintained for several minutes; in the examples of figures 3B, 4B and 5B the peak has a duration of about 5 minutes and comprises an increase followed by a decrease in the characteristic value or a decrease followed by an increase in the characteristic value. The peak value is the maximum amplitude (or respectively minimum amplitude if the peak goes below the baseline) value taken by the concentration or conductivity during the peak; in a possible embodiment the control device 301 may for instance cause, e.g. every 30 minutes, a short variation in the characteristic of the fresh dialysis liquid, for instance a change in the dialysate conductivity comprising a step variation of 1 or more mS/cm lasting 1 or more minutes (e.g. 2 to 10 minutes) with respect the base line value of the conductivity (figures 3A, 4A, 5A show these changes or peaks 10). As mentioned above according to one embodiment the control device 301 is connected to the first sensor $S_1$ and to one or both said second and third sensors $S_2$, $S_3$ and is configured for receiving, from said sensors, values taken over time by said parameter BV%(t) and by said characteristic Cd(t) in the fresh dialysis line and/or in the evacuation line. For example, the control device 301 may receive the conductivity values Cd(t) taken over time by the dialysis liquid (e.g. in the fresh dialysis line as measured by sensor $S_2$ or in the evacuation line as measured by sensor $S_3$) and the blood volume variation values BV%(t) and store these values in memory block 303. Note that instead of using measured values for the characteristic Cd(t), set values may be used. In any case, either by measuring values with one or both the second and the third sensor or by using the set values for Cd(t), the control unit knows when a peak is imposed onto the characteristic Cd(t). In general, note that the values of the parameter, namely the percent blood volume changes, are measured values detected by the first sensor; on the other hand the values of the characteristic may be either measured or set values and comprise the values taken over time by said characteristic in the fresh dialysis liquid line or in the evacuation line: the characteristic may be either the conductivity or the concentration for at least one substance (e.g. sodium or a group of electrolytes).Figures 3A, 4A, 5A show the percent change in the blood volume over time BV%(t), while figures 3B, 4B, 5B show the change over time of the dialysate conductivity Cd(t).

The values of the parameter and those of the characteristic (e.g. BV%(t) and Cd(t)) may be stored in the memory block 303 and transmitted to the monitoring apparatus 100. The monitoring apparatus receives from the machine or machines 300 part of the infrastructure 1, and for each monitored patient, the values of the parameter and those of the characteristic (e.g. BV%(t) and Cd(t)) relating to a plurality of consecutive treatments delivered to a same patient.

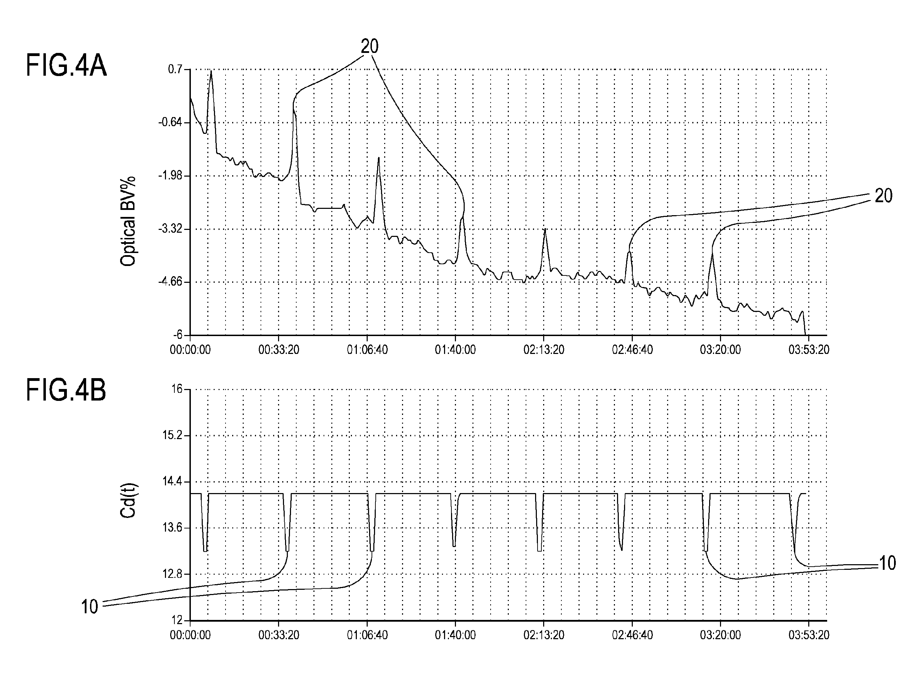

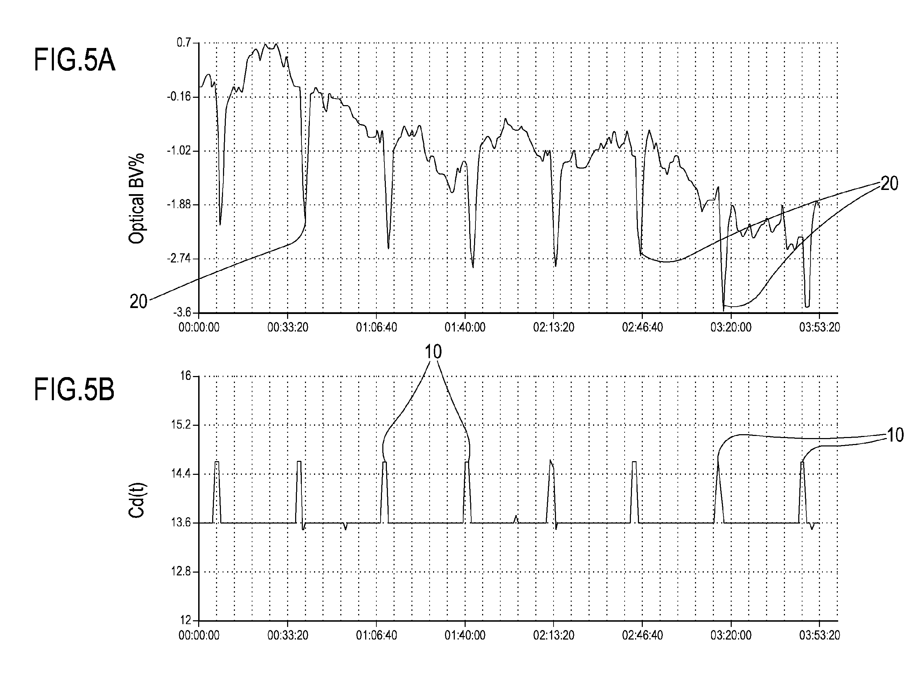

The monitoring apparatus 100, which may be located remotely with respect to the blood treatment machines of the infrastructure 1, may include an own control unit 103 a display device 102 connected to the control unit and a memory block (or memories) 101 also connected to the control unit. As an alternative, in case the monitoring apparatus is part of a blood treatment machine, the control unit of the monitoring apparatus may be a part of the blood treatment machine control device 301. In any case the control unit 101 of the monitoring apparatus is configured for establishing a communication with the control device of the extracorporeal blood treatment machine: if the control unit 101 and the control device 301 are physically distinct and one remotely located from the other, a communication therebetween may be established in a wired or wireless manner with or without intermediate unit 200. In case the control unit 101 is part of the control device 301 then there would be an internal transmission of information among blocks or tasks of the same control system. Irrespective of the specific architecture taken by infrastructure 1, the control unit 101 is configured for receiving measured values of said parameter BV%(t) which relate to a same patient and refer to a plurality of distinct treatment and consecutive sessions, and for receiving measured or set values of said characteristic Cd(t) which also relate to a same patient and refer to a plurality of distinct and consecutive treatment sessions. For instance information of the type contained e.g. in the tables of figures 3A, 4A, 5A, 3B, 4B, 5B may be received by control unit 101 which is configured to then analyze the received information and identify presence of peaks in the values taken over time by the characteristic Cd(t) during a same treatment session and of peaks in the values taken over time by the parameter BV%(t) during a same treatment session. More in detail, the control unit is configured to check whether - in presence of a peak in the values taken characteristic Cd(t) - there is a corresponding presence of a peak in the values taken by parameter BV%(t). For instance referring to the example of figures 3A, 3B the control unit identifies peaks 10 in the values taken by the characteristic Cd(t) and identifies no peaks in the values taken by the parameter BV%(t). On the other hand, referring to the example of figures 4A, 4B and in the example of figures 5A, 5B, the control unit identifies peaks 10 in the values taken by the characteristic Cd(t) and identifies corresponding, substantially simultaneous, peaks 20 in the values taken by the parameter BV%(t). In general, any method able to filter signal noise and identify changes going beyond noise level may be suitable for identifying BV%(t) peaks. Note that in the examples of figures 5A, 5B and 4A, 4B for each peak 10 in the characteristic there corresponds a substantially simultaneous peak 20 in the parameter thereby defining a pair of substantially concurrent peaks in a same treatment session: for instance figures 5A and 5B show a case where eight consecutive pairs of peaks can be identified. For the purpose of the present invention, peaks are considered substantially simultaneous and define a pair of peaks when the peak value in the parameter BV%(t) takes place within an interval of 0 to 5 minutes after the peak value in the characteristic Cd(t) in either the fresh or spent dialysis fluid line.

The control unit 101 is also programmed or configured for determining if presence of said pair of substantially concurrent peaks in a same treatment session takes place for at least two or more consecutive peaks in the values of the characteristic; if - as in the examples of figures 4A, 4B, 5A and 5B - the control unit verifies presence of a plurality of immediately consecutive pairs of substantially concurrent peaks, then the control unit establishes that a condition of recirculation took place in the concerned treatment session. In other words, in a situation as the one shown in figures 4A, 4B, 5A and 5B, the control unit would conclude that there has been recirculation in the treatment session relating to the analyzed values. Without being bound by theory, it is believed that a peak in the conductivity/concentration in the dialysis fluid - in presence of recirculation - causes a change in the optical properties of blood and thus a peak (directed in the opposed direction with respect to the peak in the dialysis liquid characteristic) is reflected BV% measure made in the blood circuit by the first sensor.

The control unit 101 is further configured for verifying if said condition of recirculation is taking place on the same patient for two or more consecutive treatment sessions: based on the outcome of said verification of said condition of recirculation the control unit 101 is configured for identifying a cause of recirculation. More in detail, the control unit establishes that the cause of recirculation is a degradation in the status of the vascular access, for instance a fistula stenosis or fibrosis, if said condition of recirculation is taking place for at least two consecutive treatment sessions on the same patient.

On the other hand, the control unit 101 establishes that the cause of recirculation is due to improperly positioned access devices, for instance access needles installed in reversed position in the vascular access, if said condition of recirculation is taking place for one isolated treatment session immediately preceded and followed by treatment sessions where said condition of recirculation is not taking place.

The step of checking the presence of a pair of simultaneous peaks comprises verifying if each pair of substantially concurrent peaks comprises peaks in opposite in direction with respect to the respective baseline values, that is: in case of a peak in the characteristic Cd(t) going above with respect to the characteristic baseline value the control unit 101 verifies a corresponding presence of a peak in the parameter value BV%(t) going below with respect to the parameter baseline value (example of figures 5A, 5B), whilst in case of a peak in the characteristic Cd(t) going below with respect to the characteristic baseline value the control unit 101 verifies a corresponding presence of a peak in the parameter value BV%(t) going below with respect to the parameter baseline value (example of figures 4A, 4B).

The control unit is thus configured to conclude that the condition of recirculation is taking place if both the following conditions are complied with:

- presence of said pair of substantially concurrent peaks in a same treatment session takes place for at least two or more consecutive peaks in the values of the characteristic;
- said pair of substantially concurrent peaks comprises peaks in opposite in direction with respect to the respective baseline values.

In other words, to have a determination of presence of recirculation the control unit makes sure that a plurality of consecutive pairs of peaks in the Cd(t) and in BV%(t) are detected wherein said peaks are oppositely oriented worth respect to the respective baselines.

As mentioned, the control unit may adopt any suitable method in order to discriminate presence of a peak.

According to one aspect of the invention, the knowledge of the presence and of the timing of peaks in the values taken over time by the characteristic Cd(t) may simply be provided by the control device 301 which is governing the preparation section 318. Alternatively, presence and timing of peaks in the values taken over time by the characteristic Cd(t) may be provided by the control unit 101 by:

- analyzing the values taken by said characteristic Cd(t) over time,
- identifying presence of a peak when the analyzed values of the characteristic Cd(t)show a sudden change with respect to the respective baseline. A change is considered by the control unit to be a sudden change in the characteristic value Cd(t) if it includes an increase or a decrease by at least 5% in the value of the characteristic taking place in a time interval of less than 5 minutes.

According to another aspect of the invention, the presence of peaks in the values taken over time by the parameter BV%(t) comprises:

- analyzing the values taken by said parameter BV%(t) over time,
- identifying presence of a peak if the analyzed values of the parameter BV%(t) show a sudden change in the parameter value with respect to the respective baseline. A change is considered to be a sudden change if it includes an increase or a decrease by at least a known threshold, e.g.10% in the value of the parameter, taking place in a time interval of less than a certain reference time, e.g. 5 minutes.

As mentioned any mathematical method capable of analyzing the signals or the values of representative of the characteristic Cd(t) or of the parameter BV%(t) may be used in order to discriminate the presence of peaks with respect to the baseline and baseline noise.

In accordance with a further aspect the control unit is configured for establishing that a condition of recirculation took place in the treatment session if:

- presence of said pair of substantially concurrent peaks occurs for at least two consecutive peaks in the same session and
- the magnitude of the peaks in the parameter is substantially constant (meaning that the peak values are stable in magnitude). For example, the magnitude of two or more immediately consecutive peaks is considered to be stable if the peak value (which is the maximum or minimum value taken by BV%(t) during the peak) of a subsequent peak in not grater (or not smaller) than 20% of the peak value relating to the immediately preceding peak.

If the control unit verifies that the parameter peaks have a stable magnitude, then the control unit is programmed to establish that there is a high probability of recirculation.

The above described method, particularly when the parameter peaks show a stable magnitude, offers good reliability in determining recirculation and in assessing the cause thereof. However, in order to further enhance reliability in the recirculation determination, the control unit 101 may also be configured to determine, a parameter relative to efficiency of the dialysis treatment, such as dialysance or clearance, or dialysis dose, or concentration for one substance (e.g. sodium) in blood of the patient under treatment. The dialysance (D) of a solute is defined as the mass of solute extracted from the blood per unit time divided by the difference between the concentration of this solute in the blood and of this solute in the dialysis liquid, on entry to the dialyzer or filter. This definition in general applies when the solute is present in the blood and in the fresh dialysis liquid (before entering the filter and contact with the blood via the semi-permeable membrane). The clearance (K) of a solute is a particular case of the dialysance of a solute. Clearance of a solute is the dialysance when the solute is present in the blood only and consequently is absent from the fresh dialysis liquid: for instance we shall speak of urea clearance and of sodium dialysance. Finally, the total dialysis dose delivered is the integral of the values of average clearance or dialysance measured over a determined time interval. The dialysis dose administered after a treatment time t can be regarded, according to the work of Sargent and Gotch, as the dimensionless ratio Kt/V, where K is the real clearance for the urea, t the elapsed treatment time, and V the volume of distribution of the urea, that is to say the total water volume of the patient (Gotch FA, Sargent SA. A mechanistic analysis of the National Cooperative Dialysis Study (NCDS). Kidney int 1985; 28: 526-34).

The dialysance or clearance of a solute may be calculated in different ways: on line in the extracorporeal circuit during the treatment or after the treatment, in-vivo during the treatment or after the treatment, once or several times by periodic samples, etcetera.

In practice - in order to in-vivo determine e.g. the dialysance or the blood conductivity or the concentration of a substance (such as Na, for example) in blood - any one of the procedures disclosed in the following publications may be adopted. Document EP 0547025 describes a method for determining dialysance and the conductivity or concentration of a substance, such as sodium, in a patient's blood subjected to a dialysis treatment. This method also makes it possible to determine the dialysance D - for example for sodium - of the blood treatment unit or dialyzer used. The method comprises the steps of circulating a first and a second dialysis liquids having different sodium concentrations in succession through the second chamber of the blood treatment unit, measuring the conductivity of the first and second dialysis liquids upstream and downstream of the dialyzer, and computing the concentration of sodium in the patient's blood (or the dialysance D of the dialyzer for sodium) from the values of the conductivity of the liquid which are measured in the first and second dialysis liquids upstream and downstream of the dialyzer. Document EP 0658352 describes another method for the in vivo determination of dialysis parameters (including dialysance, conductivity or concentration of a substance, such as sodium, in a patient's blood) which comprises the steps of: making at least a first and a second treatment liquids, having a characteristic (the conductivity, for example) associated with at least one of the parameters (the ion concentration of the blood, the dialysance D, the clearance K, Kt/V, for example) indicative of the treatment, flow in succession through the dialyzer, the value of the characteristic in the first liquid upstream of the exchanger being different from the value of the characteristic in the second liquid upstream of the dialyzer; measuring, in each of the first and second treatment liquids, two values of the characteristic, respectively upstream and downstream of the dialyzer; making a third treatment liquid flow through the dialyzer while the characteristic of the second liquid has not reached a stable value downstream of the dialyzer, the value of the characteristic in the third liquid upstream of the dialyzer being different from the value of the characteristic in the second liquid upstream of the dialyzer; measuring two values of the characteristic in the third liquid, respectively upstream and downstream of the dialyzer; and computing at least one value of at least one parameter indicative of the progress of the treatment from the measured values of the characteristic in the first, second and third treatment liquids. Another method for the in vivo determination of the dialysis parameters (including dialysance ,conductivity or concentration of a substance, such as sodium, in a patient's blood) which does not require taking measurements on blood samples is described in document EP 0920877. This method includes the steps of: making a treatment liquid flow through the exchanger, this treatment liquid having a characteristic which has an approximately constant nominal value upstream of the exchanger; varying the value of the characteristic upstream of the exchanger and then re-establishing the characteristic to its nominal value upstream of the exchanger; measuring and storing in memory a plurality of values adopted by the characteristic of the treatment liquid downstream of the exchanger in response to the variation in the value of this characteristic caused upstream of the exchanger; determining the area of a downstream perturbation region bounded by a baseline and a curve representative of the variation with respect to time of the characteristic; and computing the parameter indicative of the effectiveness of a treatment from the area of the downstream perturbation region and from the area of an upstream perturbation region bounded by a baseline and a curve representative of the variation with respect to time of the characteristic upstream of the exchanger. Of course, any other procedure adapted for the in vivo determination of dialysance, blood conductivity or concentration for one substance without blood sampling may equivalently be adopted. For instance document US 2001004523 describes a solution for continuously determining dialysance/clearance for one substance, conductivity/concentration in blood comprising the steps of: causing a succession of sinusoidal variations in the characteristic (Cd) a treatment liquid upstream of the exchanger, continuously storing in memory a plurality of values (Cdin1 ... Cdinj ... Cdinp) of the characteristic (Cd) upstream of the exchanger, measuring and continuously storing in memory a plurality of values (Cdout1 ... Cdoutj ... Cdoutp) adopted by the characteristic (Cd) downstream of the exchanger in response to the variations in the characteristic (Cd) which are caused upstream of the exchanger, computing - each time that a predetermined number of new values (Cdoutj) of the characteristic (Cd) downstream of the exchanger has been stored

- said parameter (D, Cbin, K, Kt/V) from a first series of values (Cdinj) of the characteristic (Cd) upstream of the exchanger, from a second series of values (Cdoutj) of the characteristic (Cd) downstream of the exchanger.

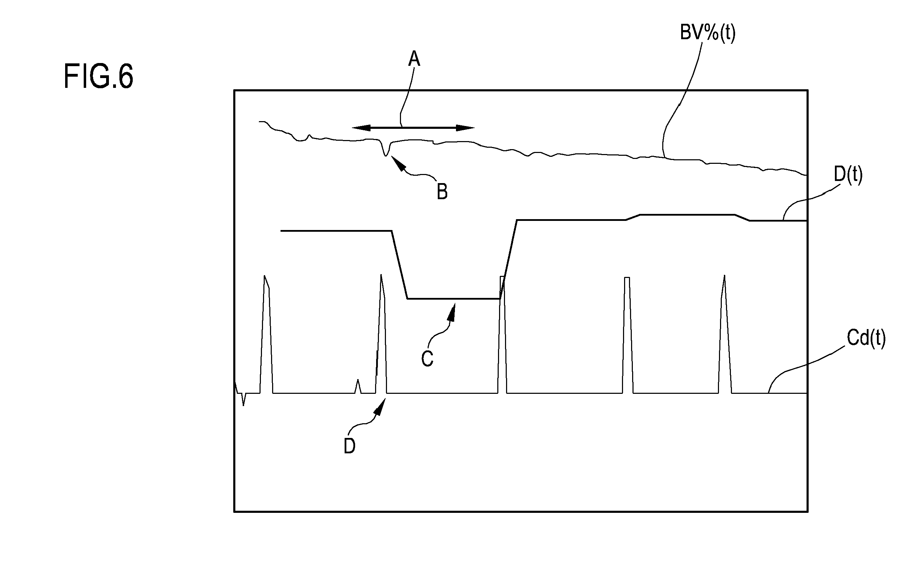

In accordance with an aspect of the present invention, the effectiveness parameter, for instance dialysance, is determined in vivo e.g. using one of the above described methods. In an embodiment, at each variation in the characteristic of the fresh dialysis liquid the control unit receives measures of the conductivity (or the concentration in one substance) in the spent dialysis fluid and calculates the in-vivo dialysance. Thus, as shown in figure 6, the in-vivo measured dialysance value is available for each pair of peaks. The control unit is configured for comparing each measured dialysance value with a threshold: if the control unit then verifies that 1) a sequence of consecutive pairs of peaks (in the characteristic and in the parameter values, as above described indicating high likelihood of recirculation) and 2) at each characteristic change the measured dialysance, relating to the same consecutive peaks in the characteristic, is below said prefixed threshold, the control unit conclude that there is recirculation and the reliability of the detection is considered to be very high. The situation of figure 6, shows an example where recirculation has been induced by momentarily reversing the blood lines such as to withdraw blood from a downstream point of the fistula and return treated blood to an upstream point of the fistula: as shown in figure 6, recirculation causes a dialysance drop, so by combining the dialysance detections with the detection of the peaks in the parameter BV%(t) and in the characteristic Cd(t), it is possible to improve the reliability of the method.

**Process**

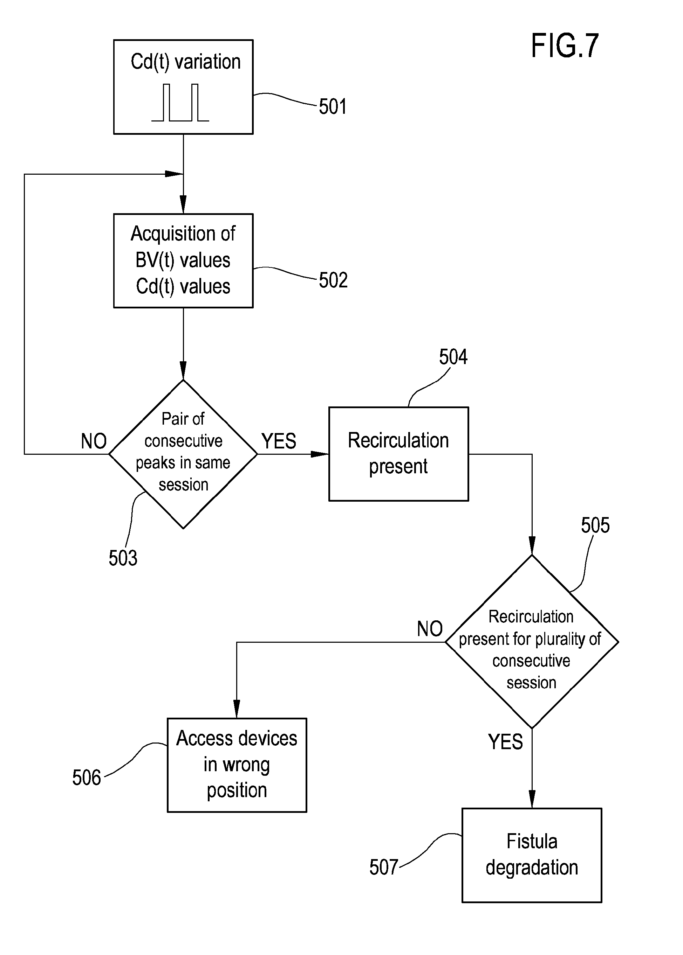

A process of monitoring a plurality of patients over a time frame covering a plurality of blood treatment sessions is now described. Reference is made to the block diagram of figure 7 where an example of a process 500 according to aspects of the invention is shown.

The process may use the above described infrastructure and, in particular, the above described monitoring apparatus.

For instance, at time intervals, an upstream variation $Cd_{in}$ is introduced to the characteristic in the fresh treatment liquid with respect to its prescription baseline thereby determining a corresponding and timely delayed downstream variation $Cd_{out}$ of the same characteristic in the spent liquid flowing in the evacuation line. The upstream variation may be created at regular, i.e. periodic, time intervals. In figure 7 this first step is identified by block 501.

As a second step 502 the conductivity values Cd(t) taken over time by the dialysis liquid and the blood volume variation values BV%(t) are acquired. The values of the parameter, namely the percent blood volume changes, are measured values detected by the first sensor; on the other hand the values of the characteristic may be either measured or set values and comprise the values taken over time by said characteristic in the fresh dialysis liquid line or in the effluent line: the characteristic may be either the conductivity or the concentration for at least one substance (e.g. sodium or a group of electrolytes) in the fresh dialysis liquid.

At step 503 the process provides for determining presence of peaks in the values taken over time by the characteristic Cd(t) during a same treatment session and presence of peaks in the values taken over time by the parameter BV%(t) during a same treatment session. More in detail, the process provides for verification as to whether - in presence of a peak in the values taken characteristic Cd(t) - there is a corresponding presence of a peak in the values of taken by parameter BV%(t). Note that in the examples of figures 5A, 5B and 4A, 4B for each peak 10 there corresponds a substantially simultaneous peak 20 thereby defining a pair of substantially concurrent peaks in a same treatment session: for instance figures 5A and 5B show a case where eight consecutive pairs of peaks can be identified.

If presence of said pair of substantially concurrent peaks in a same treatment session takes place for at least two or more consecutive peaks in the values of the characteristic (as in the examples of figures 4A, 4B, 5A and 5B) then it is concluded (step 504) that a condition of recirculation took place in the concerned treatment session. In other words, in a situation as the one shown in figures 4A, 4B, 5A and 5B, the process determines that there has been recirculation in the treatment session relating to the analyzed values.

The process also verifies, at step 505, if said condition of recirculation is taking place on the same patient for two or more consecutive treatment sessions and identifies a cause of recirculation based on the outcome of said verification of said condition of recirculation. More in detail, see step 507, the cause of recirculation is a degradation in the status of the vascular access, for instance a fistula stenosis or fibrosis, if said condition of recirculation is taking place for at least two consecutive treatment sessions on the same patient. On the other hand, see step 506, the cause of recirculation is due to improperly positioned access devices, for instance access needles installed in reversed position in the vascular access, if said condition of recirculation is taking place for one isolated treatment session immediately preceded and followed by treatment sessions where said condition of recirculation is not taking place.

The step of checking the presence of a pair of simultaneous peaks comprises verifying if each pair of substantially concurrent peaks comprises peaks in opposite in direction with respect to the respective baseline values, that is: in case of a an upwardly directed peak in the characteristic Cd(t) with respect to the characteristic baseline value it is verified that there exist a corresponding presence of a downwardly directed peak in the parameter value BV%(t) with respect to the parameter baseline value (example of figures 5A, 5B). Analogously, in case of a downwardly directed peak in the characteristic Cd(t) with respect to the characteristic baseline value it is verified that there exist a corresponding presence of an upwardly directed peak in the parameter value BV%(t) with respect to the parameter baseline value(example of figures 4A, 4B).

In practice it is concluded that a condition of recirculation took place if both the following conditions are complied with:

- presence of said pair of substantially concurrent peaks in a same treatment session takes place for at least two or more consecutive peaks in the values of the characteristic;

- said pair of substantially concurrent peaks comprises peaks in opposite in direction with respect to the respective baseline values.

The above described method, particularly when the parameter peaks show a stable magnitude, offers good reliability in determining recirculation and in assessing the cause thereof.

However, in order to further enhance reliability in the recirculation determination, the process may include the determination of a parameter relative to efficiency of the dialysis treatment, such as dialysance or clearance, or dialysis dose or concentration for one substance (e.g. sodium) in blood of the patient under treatment. In accordance with an aspect of the present invention, the effectiveness parameter, for instance dialysance, is determined in vivo e.g. using one of the above disclosed methods. In practice, the process provides for determination of dialysance at each peak of the characteristic Cd(t) and compares each measured dialysance value with a threshold: then, if

1) a sequence of consecutive pairs of peaks (in the characteristic and in the parameter values, as above described indicating high likelihood of recirculation) is detected and if
2) at each characteristic change the measured dialysance, relating to the same consecutive peaks in the characteristic, is below said prefixed threshold,

the process concludes that there is recirculation and the reliability of the detection is considered to be very high.

Finally, the above disclosed monitoring device, infrastructure and process may also be conveniently combined with the criteria disclosed in WO2008/087470 for the determination of the access score, which is a measure of the quality of the fistula (or other access to the patient cardiovascular system) in order to provide an even more robust information as to the status of degradation of the fistula.

Although the invention has been described in connection with certain practical embodiments, it is to be understood that the invention is not to be limited to the disclosed embodiments, but on the contrary, is intended to cover various modifications and equivalent arrangements included within the spirit and the scope of the appended claims.

**Claims**

1. A medical infrastructure comprising:

   at least one extracorporeal blood treatment machine having:

   - an extracorporeal blood circuit (330) with a blood treatment unit (312) provided with at least a first chamber (311) and at least a second chamber (313) separated from one another by a semipermeable membrane (314);
- a dialysate circuit (340) comprising:

a dialysis line (315) configured to convey fresh dialysis fluid to the second chamber (313),
a fluid evacuation line (316) configured to discharge spent dialysis fluid exiting from the second chamber (313), and
a fluid delivery section (318) connected to the dialysis line (315) and configured for adjusting at least one physical or chemical characteristic (Cd(t)) in the fresh dialysis fluid;

- at least one first sensor ($S_1$) connected to the extracorporeal blood circuit (300) and configured for measuring values of a parameter (BV%(t)) - relating to the change of blood volume in the blood circulating in the extracorporeal blood circuit - measured during the course of extracorporeal blood treatment;
- a control device (301) connected to said first sensor ($S_1$) and configured for receiving from said first sensor ($S_1$) values taken over time by said parameter, the control device being also connected to said fluid delivery section (318) and configured for controlling said fluid delivery section to adjust said characteristic Cd(t);

a monitoring apparatus (100) comprising a control unit (103) configured for:

- receiving measured values of said parameter (BV%(t)), said measured values of said parameter relating to a same patient and referring to a plurality of distinct treatment sessions;
- receiving values of said characteristic (Cd(t)), said values of said characteristic relating to said same patient and referring to the same plurality of distinct treatment sessions;
- checking whether - in presence of a peak in the values taken by the characteristic (Cd(t))- there is a corresponding presence of a peak in the values of the parameter (BV%(t))thereby defining a pair of substantially concurrent peaks in a same treatment session;
- establishing that a condition of recirculation took place in the treatment session if presence of said pair of substantially concurrent peaks occurs for at least two - optionally at least three - consecutive peaks in the values of the characteristic; then
- verifying if said condition of recirculation is taking place on the same patient for at least two consecutive treatment sessions;
- identifying a cause of recirculation based on the outcome of said verification of said condition of recirculation.

2. A medical infrastructure according to claim 1, wherein establishing comprises the following sub-steps:

- determining presence of peaks in the values taken over time by the characteristic (Cd(t)) during the same treatment session;
- determining presence of peaks in the values taken over time by the parameter (BV%(t)) during said same treatment session;
- determining whether presence of said pair of substantially concurrent peaks in the same treatment session takes place for at least two - optionally at least three - consecutive peaks in the values of the characteristic, and in the case where presence of said pair of substantially concurrent peaks occurs for at least two - optionally at least three - consecutive peaks in the values of the characteristic, concluding that a condition of recirculation took place in the treatment session.

3. A medical infrastructure according to any one of the preceding claims, wherein the monitoring apparatus (100)is remotely located and communicatively connected to the extracorporeal blood treatment machine (300) and wherein the control unit (101) is configured for establishing a communication with the control device (301) of the extracorporeal blood treatment machine (300) and for receiving said values of the parameter and of the characteristic from said control device.

4. A medical infrastructure according to any one of the preceding claims, wherein the control device (301) is configured for:

O causing fresh treatment liquid to flow in the dialysis line (315) to the secondary chamber (4) with said characteristic being at a baseline (Cdset) set value which is either constant or varying in a known manner over time;
O causing spent liquid to flow out of the second chamber (313) into the evacuation line (316);
O causing a peak in the characteristic (Cd(t)) by imposing an upstream peak (Cdin) to said characteristic in the fresh treatment liquid with respect to said prescription baseline thereby determining a corresponding and timely delayed downstream peak (Cdout) of the same characteristic in the spent liquid flowing in the evacuation line (13).

5. A medical infrastructure according to any one of the preceding claims, wherein the control unit (101) of the monitoring apparatus (100) is configured for:

- receiving measured or set values of said characteristic (Cd(t)), said values of said characteristic relating to the same patient and referring to a plurality of distinct treatment sessions;
- checking presence of peaks in the characteristic (Cd(t)) by analysing either the measured or the set values of said characteristic (Cd(t)).

6. A medical infrastructure according to any one of the preceding claims, wherein identifying a cause of recirculation comprises:

- establishing that the cause of recirculation is a degradation in the status of the vascular access, optionally a fistula stenosis or fibrosis, if said condition of recirculation is verified to take place for at least two consecutive treatment sessions on the same patient, and
- establishing that the cause of recirculation is due to improperly positioned access devices, optionally access devices installed in reversed position in the vascular access, if said condition of recirculation is verified to take place for one isolated treatment session immediately preceded and followed by treatment sessions where said condition of recirculation is verified not to take place.

7. A medical infrastructure according to any one of the preceding claims 5 or 6, wherein the extracorporeal blood treatment machine (300) comprises at least one of:

- a second sensor ($S_2$) configured for measuring values of the physical or chemical characteristic (Cd(t)) of the fresh dialysis liquid flowing in the dialysis line during the course of the extracorporeal blood treatment, and
- a third sensor ($S_3$) configured for measuring values of the physical or chemical characteristic (Cd(t)) of the spent liquid flowing in the evacuation line during the course of the extracorporeal blood treatment;

wherein receiving measured values of said characteristic (Cd(t)) comprises receiving the values taken over time by said characteristic which comprises the conductivity or the concentration for at least one substance in the fresh or in the spent dialysis liquid.

8. A medical infrastructure according to any one of the preceding claims, wherein said step of checking comprises verifying if each pair of substantially concurrent peaks comprises peaks in opposite in direction with respect to the respective baseline values.

9. A medical infrastructure according to any one of the preceding claims, wherein said step of determining presence of peaks in the values taken over time by the characteristic comprises:

- analyzing the values taken by said characteristic (Cd(t)) over time,
- identifying presence of a peak when the analyzed values of the characteristic (Cd(t)) show a sudden change with respect to the respective baseline, wherein a sudden change in the characteristic value is an increase or a decrease by at least 5% in the value of the characteristic taking place in a time interval of less than 5 minutes; and

wherein said step of determining presence of peaks in the values taken over time by the parameter (BV%(t)) comprises:

- analyzing the values taken by said parameter over time,
- identifying presence of a peak if the analyzed values of the parameter show a sudden change in the parameter value (BV%(t) with respect to the respective baseline, wherein a sudden change is an increase or a decrease by at least 10% in the value of the parameter taking place in a time interval of less than 5 minutes.

10. A medical infrastructure according to any one of the preceding claims, wherein the control unit (101) is configured for establishing that a condition of recirculation took place in the treatment session if:

- presence of said pair of substantially concurrent peaks occurs for at least two consecutive peaks in the same session and
- the magnitude of the peak values in said two consecutive peaks in the parameter values is substantially constant.

11. A medical infrastructure according to any one of the preceding claims, wherein the control unit (101) is configured for:

- in-vivo determining, at each peak in the characteristic (Cd(t)), values of a parameter (D(t)) relative to efficiency of the dialysis treatment,
- comparing the values of the efficiency parameter (D(t)) against a respective threshold,
- concluding that a condition of recirculation took place in the treatment session if:

presence of a sequence of consecutive pairs of substantially concurrent peaks is detected and
the in-vivo determined value of the efficiency parameter, relating to one or both consecutive peaks in the characteristic, is below said prefixed threshold.

12. A medical monitoring apparatus for surveillance of patients submitted to a plurality of extracorporeal treatment sessions executed by at least one extracorporeal blood treatment machine, the monitoring apparatus comprising:

- at least one memory block configured for storing:

○ measured values of a parameter (BV%(t)) relating to the change of blood volume, in the blood circulating in an extracorporeal blood circuit of said machine, as measured during the course of blood treatment, said measured values of said parameter relating to a same patient and referring to a plurality of distinct treatment sessions;
○ measured or set values of a physical or chemical characteristic (Cd(t)) of the dialysis liquid as set or, respectively, as measured during the course of the extracorporeal blood treatment, said values of said characteristic relating to said same patient and referring to said plurality of distinct treatment sessions;

- at least a control unit (101) connected to the memory block and configured for:

○ determining presence of peaks in the values taken over time by the characteristic (Cd(t)) during a same treatment session;
○ determining presence of peaks in the values taken over time by the parameter (BV%(t))during the same treatment session;
○ checking whether - in presence of a peak in the values of the characteristic - there is a corresponding presence of a peak in the values of the parameter thereby defining a pair of substantially concurrent peaks in a same treatment session;
○ determining whether presence of said pair of substantially concurrent peaks in a same treatment session takes place for at least two - optionally at least three - consecutive peaks in the values of the characteristic, and

○ if presence of said pair of substantially concurrent peaks occurs for at least two - optionally at least three - consecutive peaks in the values of the characteristic, then establishing that a condition of recirculation took place in the treatment session;
○ verifying if said condition of recirculation is taking place on the same patient for at least two consecutive treatment sessions;
○ identifying a cause of recirculation based on the outcome of said verification on said condition of recirculation.

13. A medical monitoring apparatus according to claim 12 further comprising a communication interface connected to the control unit, wherein the control unit is configured for establishing a communication with a control device of the extracorporeal blood treatment machines via said communication interface.

14. A medical monitoring apparatus according to any one of the preceding claims 12 or 13, wherein the control unit (101) of the monitoring apparatus (100) is configured for:

- receiving measured or set values of said characteristic (Cd(t)), said values of said characteristic relating to the same patient and referring to a plurality of distinct treatment sessions;
- checking presence of peaks in the characteristic (Cd(t)) by referring to either the measured or the set values of said characteristic (Cd(t)); and
wherein identifying a cause of recirculation comprises:
- establishing that the cause of recirculation is a degradation in the status of the vascular access, optionally a fistula stenosis or fibrosis, if said condition of recirculation is verified to take place for at least two consecutive treatment sessions on the same patient, and
- establishing that the cause of recirculation is due to improperly positioned access devices, optionally access devices installed in reversed position in the vascular access, if said condition of recirculation is verified to take place for one isolated treatment session immediately preceded and followed by treatment sessions where said condition of recirculation is verified not to take place.

15. A medical monitoring apparatus according to any one of the preceding claims from 12 to 14, wherein said step of checking comprises verifying if each pair of substantially concurrent peaks comprises peaks in opposite in direction with respect to the respective baseline values.

**16.** A medical monitoring apparatus according to any one of the preceding claims from 12 to 16, wherein the control unit (101) is configured for establishing that a condition of recirculation took place in the treatment session if:

- presence of said pair of substantially concurrent peaks occurs for at least two consecutive peaks in the same session and
- the magnitude of the two consecutive peaks in the parameter values is substantially constant.

**17.** A medical monitoring apparatus according to any one of the preceding claims from 12 to 16, wherein the control unit (101) is configured for:

- in-vivo determining, at each peak in the characteristic (Cd(t)), values of a parameter (D(t)) relative to efficiency of the dialysis treatment,
- comparing the values of the efficiency parameter (D(t)) against a respective threshold,
- concluding that a condition of recirculation took place in the treatment session if:

  presence of a sequence of consecutive pairs of substantially concurrent peaks is detected and
  the in-vivo determined value of the efficiency parameter, relating to one or both consecutive peaks in the characteristic, is below said prefixed threshold.

**18.** An extracorporeal blood treatment machine comprising the medical monitoring apparatus according to anyone of preceding claims from 12 to 17.

**19.** A method of surveillance of patients submitted to a plurality of extracorporeal treatment sessions executed by at least one extracorporeal blood treatment machine, the method of surveillance comprising:

- storing measured values of a parameter (BV%(t)) relating to the change of blood volume in the blood circulating in an extracorporeal blood circuit of said machine as measured during the course of blood treatment, said measured values of said parameter relating to a same patient and referring to a plurality of distinct treatment sessions;
- storing measured or set values of a physical or chemical characteristic (Cd(t)) of the dialysis liquid as set or, respectively, as measured during the course of the extracorporeal blood treatment, said values of said characteristic relating to said same patient and referring to said plurality of distinct treatment sessions;
- determining presence of peaks in the values taken over time by the characteristic (Cd(t)) during a same treatment session;
- determining presence of peaks in the values taken over time by the parameter (BV%(t)) during a same treatment session;
- checking whether - in presence of a peak in the values of the characteristic - there is a corresponding presence of a peak in the values of the parameter thereby defining a pair of substantially concurrent peaks in a same treatment session;
- determining whether presence of said pair of substantially concurrent peaks in a same treatment session takes place for at least two - optionally at least three - consecutive peaks in the values of the characteristic, and
- if presence of said pair of substantially concurrent peaks occurs for at least two - optionally at least three - consecutive peaks in the values of the characteristic, then establishing that a condition of recirculation took place in the treatment session;
- verifying if said condition of recirculation is taking place on the same patient for at least two consecutive treatment sessions;
- identifying a cause of recirculation based on the outcome of said verification on said condition of recirculation.

**20.** A method according to claim 19, wherein identifying a cause of recirculation comprises:

- establishing that the cause of recirculation is a degradation in the status of the vascular access, optionally a fistula stenosis or fibrosis, if said condition of recirculation is verified to take place for at least two consecutive treatment sessions on the same patient, and
- establishing that the cause of recirculation is due to improperly positioned access devices, optionally access devices installed in reversed position in the vascular access, if said condition of recirculation is verified to take place for one isolated treatment session immediately preceded and followed by treatment sessions where said condition of recirculation is verified not to take place.

FIG.1

FIG.2

EP 2 792 377 A1

## FIG.3A

## FIG.3B

FIG.4A

FIG.4B

FIG.5A

FIG.5B

EP 2 792 377 A1

FIG.6

A

B

C

D

BV%(t)

D(t)

Cd(t)

EP 2 792 377 A1

FIG.7

501 — Cd(t) variation

502 — Acquisition of BV(t) values Cd(t) values

503 — Pair of consecutive peaks in same session

504 — Recirculation present

505 — Recirculation present for plurality of consecutive session

506 — Access devices in wrong position

507 — Fistula degradation

Europäisches
Patentamt

European
Patent Office

Office européen
des brevets

**EUROPEAN SEARCH REPORT**

Application Number

EP 13 16 3728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | L MERCADAL, B COEVOET, N ALBADAWY, S HACINI, B BENE, G DERAY, T PETITCLERC: "Analysis of the optical concentration curve to detect access recirculation", KIDNEY INTERNATIONAL, vol. 69, no. 4, 2 February 2006 (2006-02-02), pages 769-771, XP008164511, ISSN: 0085-2538, DOI: 10.1038/sj.ki.5000154 * the whole document * | 1-20 | INV. A61M1/16 A61M1/36 |
| A,D | WO 2008/087470 A1 (GAMBRO LUNDIA AB [SE]; BENE BERNARD [FR]) 24 July 2008 (2008-07-24) * abstract; figures * * pages 7-13 * | 1-20 | |
| A | US 2008/097272 A1 (DANIEL PIA [DE] ET AL) 24 April 2008 (2008-04-24) * the whole document * | 1-20 | |
| A | US 2012/298581 A1 (WEHMEYER WOLFGANG [DE] ET AL) 29 November 2012 (2012-11-29) * the whole document * | 1-20 | TECHNICAL FIELDS SEARCHED (IPC) A61M |
| A | US 2009/054822 A1 (MURAKAMI TOMOYA [JP] ET AL) 26 February 2009 (2009-02-26) * the whole document * | 1-20 | |

The present search report has been drawn up for all claims

| Place of search | Date of completion of the search | Examiner |
|---|---|---|
| The Hague | 29 August 2013 | Kaden, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 16 3728

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2013

| Patent document cited in search report | | Publication date | Patent family member(s) | | Publication date |
|---|---|---|---|---|---|
| WO 2008087470 | A1 | 24-07-2008 | AU | 2007344229 A1 | 24-07-2008 |
| | | | AU | 2008206758 A1 | 24-07-2008 |
| | | | CA | 2675096 A1 | 24-07-2008 |
| | | | CA | 2675383 A1 | 24-07-2008 |
| | | | CN | 101583955 A | 18-11-2009 |
| | | | EP | 2122519 A1 | 25-11-2009 |
| | | | EP | 2122523 A2 | 25-11-2009 |
| | | | FR | 2911417 A1 | 18-07-2008 |
| | | | KR | 20090101305 A | 24-09-2009 |
| | | | US | 2010105990 A1 | 29-04-2010 |
| | | | US | 2010145250 A1 | 10-06-2010 |
| | | | WO | 2008087470 A1 | 24-07-2008 |
| | | | WO | 2008087528 A2 | 24-07-2008 |
| US 2008097272 | A1 | 24-04-2008 | CN | 101098721 A | 02-01-2008 |
| | | | DE | 102005001051 A1 | 20-07-2006 |
| | | | EP | 1835950 A1 | 26-09-2007 |
| | | | JP | 4546544 B2 | 15-09-2010 |
| | | | JP | 2008526329 A | 24-07-2008 |
| | | | US | 2008097272 A1 | 24-04-2008 |
| | | | WO | 2006072271 A1 | 13-07-2006 |
| US 2012298581 | A1 | 29-11-2012 | DE | 102011102962 A1 | 29-11-2012 |
| | | | US | 2012298581 A1 | 29-11-2012 |
| | | | WO | 2012159734 A1 | 29-11-2012 |
| US 2009054822 | A1 | 26-02-2009 | CN | 101784291 A | 21-07-2010 |
| | | | EP | 2191856 A1 | 02-06-2010 |
| | | | JP | 4573860 B2 | 04-11-2010 |
| | | | JP | 2009045307 A | 05-03-2009 |
| | | | US | 2009054822 A1 | 26-02-2009 |
| | | | WO | 2009025084 A1 | 26-02-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5685989 A
- US 5595182 A
- US 5453576 A
- US 5510716 A
- US 5510717 A
- US 5312550 A
- EP 928614 A

- WO 0024440 A
- WO 2008087470 A
- EP 0547025 A
- EP 0658352 A
- EP 0920877 A
- US 2001004523 A

**Non-patent literature cited in the description**

- **SULLIVAN ; MERCADAL ; COEVOET ; ALBADAWY ; HACINI ; BENE ; DERAY ; PE-TITELERC.** Analysis of the optical concentration curve to detect access recirculation. *Kidney Int.,* 2006, vol. 69 (4), 769-7

- **GOTCH FA ; SARGENT SA.** A mechanistic analysis of the National Cooperative Dialysis Study (NCDS. *Kidney int,* 1985, vol. 28, 526-34